# DEMANDE DE BREVET EUROPEEN

(11) **EP 1 891 891 A1**
(43) Date de publication de la demande: **27.02.2008**
(21) Numéro de dépôt: 06119240.7
(22) Date de dépôt: 21.08.2006
(51) Int. Cl.: A61B 5/00

(54) **DIAPHANOSCOPE A USAGE MEDICAL**

(71) Demandeur: Université de Lausanne, 1015 Lausanne (CH); Haute Ecole d'Ingénieurs et de Gestion du Canton, 1400 Yverdon-les-Bains (CH)
(72) Inventeur: Horisberger, Beat, 1025, St-Sulpice (CH); Tanniger, Sébastien, 1040, Echallens (CH)
(74) Mandataire: Grosfillier, Philippe

(57) **Abrégé**

L'invention concerne un diaphanoscope à usage médical pour l'identification et l'analyse d'ecchymoses sous-cutanées invisibles à l'oeil nu, diaphanoscope comprenant une source de lumière blanche annulaire, des moyens pour disposer ladite source au contact de la peau et des moyens d'analyse de la lumière transmise au travers de la peau, lesdits moyens d'analyse de la lumière étant adaptés pour être reliés à un système de traitement informatisé et sont disposés en un endroit se confondant avec l'axe de symétrie de la source de lumière annulaire.

L'invention concerne l'utilisation de l'appareil précité ainsi qu'une méthode de diaphanoscopie pour l'analyse et l'identification d'ecchymoses sous-cutanées invisibles à l'oeil nu.

## Description

### Domaine de l'invention

La présente invention concerne l'analyse de tissus biologiques par transmission d'ondes électromagnétiques au travers de ceux-ci.

### Etat de la technique

La diaphanoscopie est utilisée dans le domaine médical depuis plusieurs années.
A titre d'exemple, on peut citer le brevet américain US 4,616,657 qui décrit un appareil de diaphanoscopie pour l'analyse de tissus humains, en particulier pour le dépistage du cancer du sein.
Les demandes de brevet européens EP 0 627 620 A1 et EP 0 710 832 A1 décrivent également des méthodes et des appareils pour mesurer les propriétés de diffusion et d'absorption dans un milieu diffusif tel que le tissu humain.

La diaphanoscopie est par ailleurs utilisée dans l'identification d'ecchymoses sous-cutanées invisibles à l'oeil nu (B. Horisberger, T. Krompecher, "Forensic diaphanoscopy: how to investigate invisible subcutaneous hematomas on living subjects", Int. J Legal Med, 1000 :73-78, Springer Verlag, 1997).
A cet effet, on applique une source de lumière blanche, p.ex. d'env. 1 W, sur la peau. En présence d'une ecchymose sous-cutanée, une partie de la lumière est absorbée, ce qui conduit à une diminution de la diffusion de la lumière dans les téguments et le tissu sous-cutané.

Ce phénomène se traduit concrètement par :
1. Une diminution du halo lorsque la lampe se trouve en regard d'une ecchymose.
2. Une asymétrie du halo lorsque la lampe se trouve en regard du bord de l'ecchymose.
3. Une tache sombre, lorsque l'ecchymose est de petite taille (< rayon du halo).

La diaphanoscopie est très utile dans le cadre d'investigations médico-légales en raison de sa grande sensibilité, sa haute spécificité, de la possibilité de diagnostiquer d'infimes ecchymoses échappant aux investigations d'imageries médicales. De plus elle est non invasive, maniable et représente un coût d'investigation modéré. Elle est particulièrement intéressante dans les cas de suspicion de maltraitance d'enfant, lors d'examen de personnes à peau foncée où les ecchymoses peuvent se confondre avec la couleur de la peau, ou encore chez des personnes obèses, où les ecchymoses profondes n'apparaissent pas ou que très tardivement à la surface de la peau. L'expérience des inventeurs de la présente demande a montré que la diaphanoscopie a été parfaitement adoptée par les tribunaux, où elle a joué dans certains cas un rôle déterminant dans la procédure d'enquêtes pénales et de condamnations.

Toutefois, la méthode actuelle est très dépendante de la formation et de l'expérience de l'investigateur. En effet, seule une personne au bénéfice d'une solide expérience professionnelle peut utiliser efficacement la diaphanoscopie pour l'identification d'ecchymoses sous-cutanées invisibles à l'oeil nu.

Il existe donc un besoin d'avoir une méthode d'investigation complémentaire à l'examen clinique qui permettrait de diagnostiquer, voire d'exclure des ecchymoses sous-cutanées, avec un taux de fiabilité plus élevé. En outre, il existe un besoin de pouvoir mieux caractériser les ecchymoses, p.ex. de déterminer leur date d'apparition, leur(s) cause(s), leurs dimensions, leur forme, etc...

### Exposé général de l'invention

Le problème que la présente invention se propose de résoudre réside dans une amélioration de l'analyse qualitative qui existe actuellement pour identifier et
caractériser des ecchymoses sous-cutanées invisibles à l'oeil nu.

Dans l'invention, la solution du problème précité consiste en un diaphanoscope à usage médical pour l'identification et l'analyse d'ecchymoses sous-cutanées invisibles à l'oeil nu, diaphanoscope comprenant une source de lumière blanche annulaire, des moyens pour disposer ladite source au contact de la peau et des moyens d'analyse de la lumière transmise au travers de la peau, lesdits moyens d'analyse de la lumière étant adaptés pour être reliés à un système de traitement informatisé et sont disposés en un endroit se confondant avec l'axe de symétrie de la source de lumière annulaire.

Les moyens d'analyse de la lumière sont de préférence constitués d'une caméra vidéo.

Selon une variante de l'invention, la source de lumière annulaire est disposée à une extrémité d'un élément cylindrique creux, l'autre extrémité du dit élément cylindrique étant fixée aux dits moyens d'analyse de la lumière.
Dans un mode de réalisation de l'invention, le diaphanoscope comprend des moyens pour corriger l'émission de la lumière émise par rapport à l'épaisseur du tissu sous-cutané

Alternativement ou en outre, le diaphanoscope comprend des moyens pour corriger l'émission de la lumière émise par rapport à la pigmentation de la peau.

Alternativement ou en outre, le diaphanoscope comprend des moyens pour exclure des structures correspondant à du sang intravasculaire.

Alternativement ou en outre, le diaphanoscope comprend des moyens pour analyser la densité et la démarcation des ecchymoses.

Alternativement ou en outre, le diaphanoscope comprend des moyens pour faire varier la ou les longueurs d'ondes utilisée(s).
L'invention concerne également l'utilisation d'un diaphanoscope tel que défini précédemment, l'utilisation comprenant une étape lors de laquelle on applique une source de lumière blanche de forme annulaire.

Dans une variante de l'invention, l'utilisation comprend une étape où l'on mesure l'épaisseur du tissu sous-cutané préalablement à l'analyse de la lumière transmise à travers la peau et une étape où l'on introduit des données relatives à l'épaisseur de la peau dans le système de traitement informatisé.

Dans une autre variante de l'invention, l'utilisation comprend une étape où l'on détermine la pigmentation préalablement à l'analyse de la lumière transmise à travers la peau et une étape où l'on introduit des données relatives à la pigmentation de la peau dans le système de traitement informatisé.

Dans une autre variante de l'invention, l'utilisation comprend une étape où l'on identifie des structures correspondant à du sang intravasculaire.

Dans une autre variante de l'invention, l'utilisation comprend une étape d'analyse de la densité et de la démarcation des ecchymoses.

L'invention concerne enfin une méthode d'analyse de la peau par diaphanoscopie pour l'identification d'ecchymoses sous-cutanées invisibles à l'oeil nu, ladite méthode comprenant l'analyse de la lumière blanche qui traverse la peau, caractérisée par le fait que l'on applique sur la peau une source de lumière de forme annulaire.

Le fait d'utiliser une source de lumière annulaire présente plusieurs avantages. En effet, la projection d'une source de lumière annulaire génère un halo diffusif coaxial par rapport à la source de la lumière; le centre du halo étant sombre, le contraste du halo diffusif est par conséquent plus marqué, ce qui facilite son examen.
En outre, la source annulaire permet d'exposer de manière axisymétrique le pourtour des lésions à étudier.

Par ailleurs, l'intérieur de l'anneau peut être avantageusement occupé par les moyens d'analyse de la lumière, p.ex. une caméra vidéo.

Les différences (avantages) entre l'objet de l'invention et l'état de la technique sont résumées dans le tableau ci-dessous :

| **Critères** | **Etat de la technique** | **Invention** | **Remarques** |
|---|---|---|---|
| Documentation des constatations effectuées | Difficilement réalisable | Directement intégrée | Une documentation permet d'obtenir une traçabilité des lésions recherchées, ce qui est important comme moyen de preuve devant la justice |
| Diagnostic | Subjectif, (dépendant la fortement de la formation et de l'expérience de l'examinateur) | Objectif (basé sur des données numériques, matrices) | Le diagnostic est ainsi rendu plus fiable (augmentation de la sensibilité et de la spécificité), indépendant de la formation et de l'expérience de l'examinateur |
| Influence de l'épaisseur de la couche sous-cutanée (dépend de la corpulence et de la topographie corporelle) | Dépendant | Indépendant | L'indépendance par rapport à la corpulence et à la topographie corporelle augmente la fiabilité du système et la convivialité d'utilisation |
| Influence de la pigmentation cutanée | Dépendant | Indépendant | L'indépendance par rapport à la pigmentation cutanée augmente la fiabilité du système et la convivialité d'utilisation |
| Influence du sang intra vasculaire (vaisseaux sous-cutanés) | L'examinateur doit les exclure « manuellement », de façon subjective | Exclusion automatique | Diminution des faux positifs et meilleure convivialité |
| Datation de l'ecchymose | Impossible | Possible, de façon fiable | Même la couleur d'une ecchymose visible à l'oeil nu ne permet pas de dater l'ecchymose de façon fiable |

Le dispositif selon l'invention permet d'obtenir une assistance plus fiable au diagnostic, et une documentation, comme moyen de preuve face à la justice est plus aisée, car les images peuvent être insérées dans les rapports d'expertise. Le but final étant de faciliter le diagnostic de signes de violence sur les personnes vivantes.

### Exposé détaillé de l'invention

L'invention est décrite plus en détail ci-après au moyen d'exemples illustrés par les figures suivantes :
**FIG. 1** est un schéma illustrant le principe de mesure de la présente invention ;
**FIG. 2** est un croquis de la partie source de lumière et de la pénétration de la lumière à travers différentes couches du tissu humain ;
**FIG. 3** est un schéma bloc de la présente invention ;
**FIG. 4** est un schéma bloc du logiciel d'assistance au diagnostique utilisé dans la présente invention ;
**FIG. 5** représente une variante du dispositif selon l'invention.

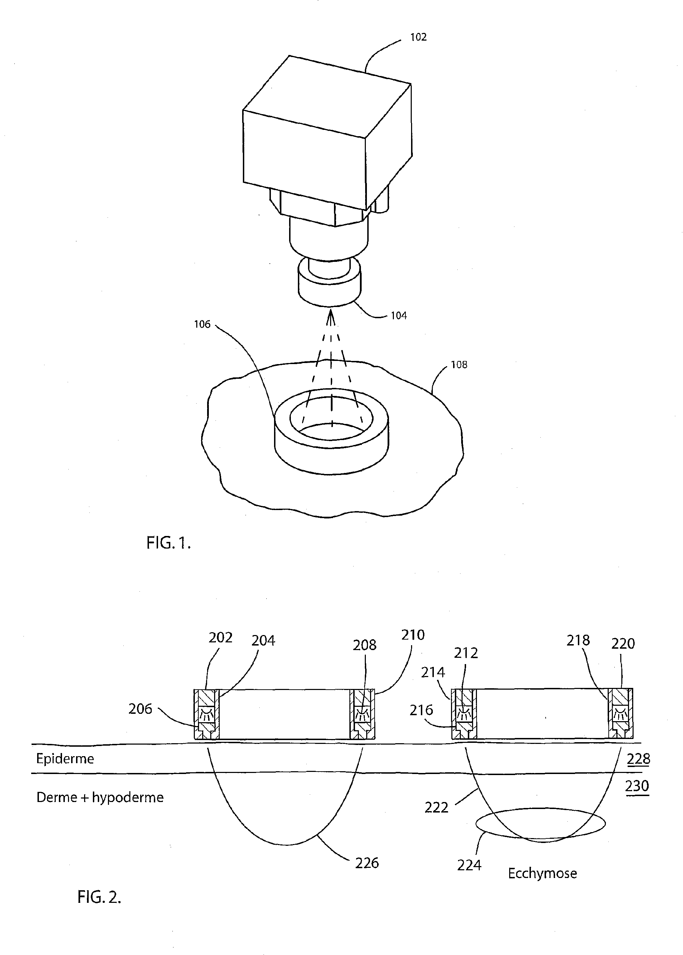

La **FIG.1** illustre un dispositif simplifié selon la présente invention, le dispositif comprend une caméra couleur **102** munie d'un objectif **104** et d'une source de lumière annulaire **106.** La zone du corps à diagnostiquer est représentée par **108.**

La **FIG.2** la source de lumière annulaire est composée de différents anneaux métalliques **204 218, 210 214** et **202 220.** La dite source de lumière est constituée de plusieurs diodes électroluminescentes **208 212** (désignée ci après par DEL) émettant un spectre de longueurs d'onde correspondant à la lumière blanche. Un anneau en matière polymère translucide **206 216** diffuse la lumière dans le derme **228** et l'hypoderme **230** pour obtenir un éclairement uniforme de la zone à analyser **224 226 .**

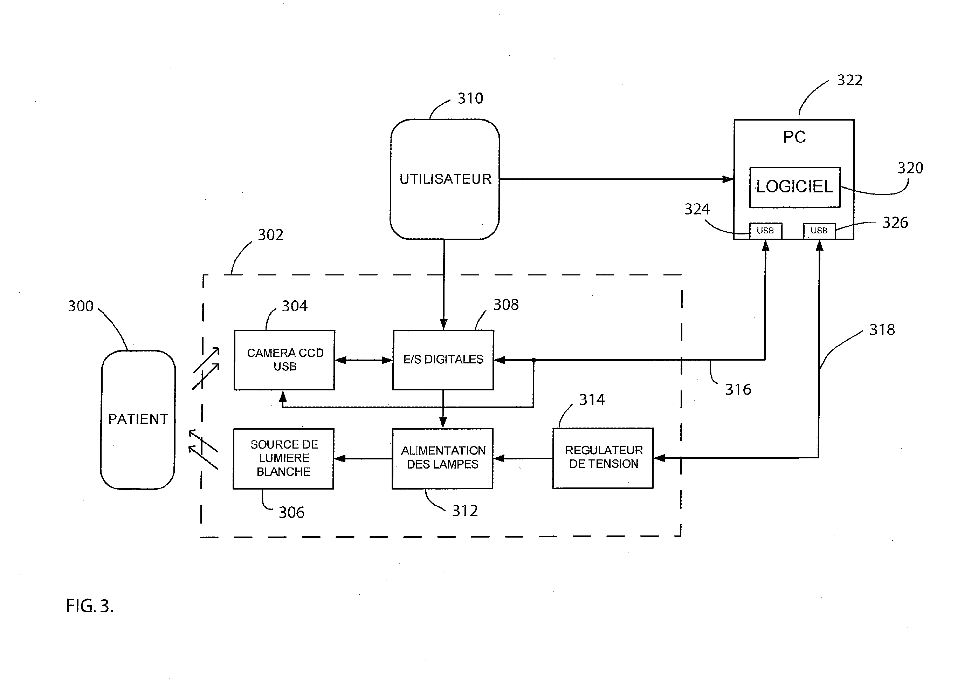

La **FIG.3** illustre le schéma bloc de la présente invention. L'ordinateur personnel de type PC **322** exécute le logiciel d'assistance au diagnostic **320.** Dans le diaphanoscope **302** les deux ports USB **324** et **326** alimentent la caméra **304,** les entrées sorties digitales **308** respectivement le régulateur de tension **314.** L'intensité d'éclairement de la source de lumière blanche **306** est gérée par l'alimentation **312** qui est pilotée par les entrées sorties digitales **308,** qui sont elles mêmes pilotées par le dit logiciel **320.** L'interaction de l'utilisateur sur le système est gérée par les entrées sorties digitales **308.** Lors de prises de mesures, la source de lumière blanche **306** est enclenchée par le dit logiciel **320,** la lumière incidente retournée par le tissu du patient **300** est enregistrée par la caméra **304** qui va envoyer les informations au dit logiciel **320** par le bus USB **316.**

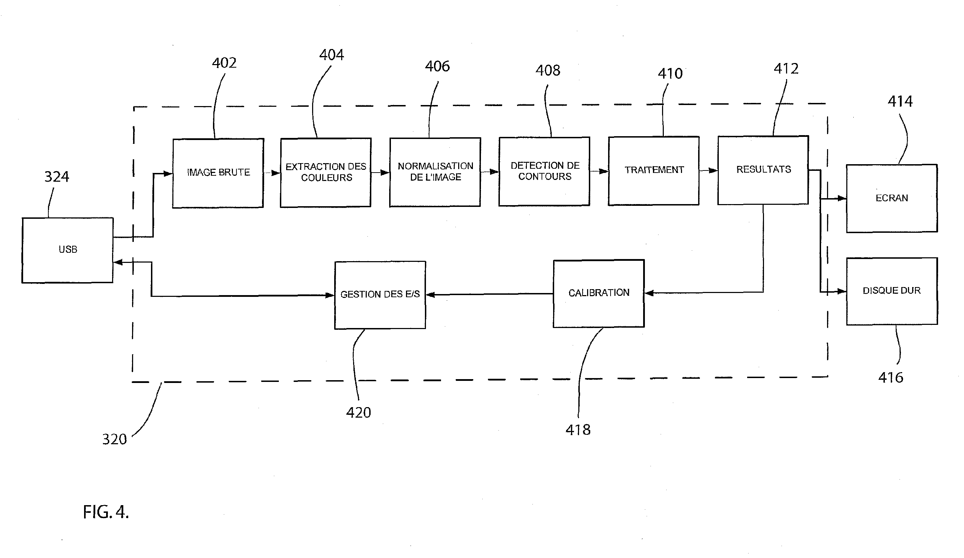

**FIG.4** illustre le schéma bloc du logiciel d'assistance au diagnostic **320.** L'image brute **402** provenant de la caméra **304** par le biais du bus USB **316.** L'extraction des couleurs est ensuite réalisée par **404.** La normalisation **406** est nécessaire pour annuler l'effet non linéaire de la diffusion de la lumière dans la peau. La détection des contours est effectuée par **408,** le traitement **410** sur les images traitées va donner les résultats **412.** Une fois les résultats obtenus, ils sont affichés sur l'écran **414** de l'ordinateur personnel de type PC **322** et archivés sur le disque dur **416.** Les résultats **412** peuvent conduire à un processus de calibration **418,** qui va agir sur la gestion des entrées sorties digitales **420.**

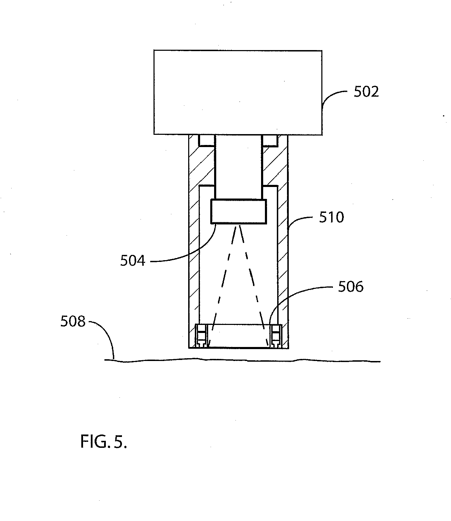

**FIG.5** illustre un variante du dispositif de l'invention comportant un tube **510** entre l'objectif **504** de la caméra **502** et la source de lumière annulaire **506** illuminant la zone du corps à diagnostiquer est représentée par **508.**

Avantageusement, le dispositif peut utiliser une source étendue capable d'exposer le pourtour des lésions à étudier.

Bien évidemment, l'invention ne se limite pas aux exemples illustrés discutés ci-dessus.

## Revendications

1. Diaphanoscope à usage médical pour l'identification et l'analyse d'ecchymoses sous-cutanées invisibles à l'oeil nu, diaphanoscope comprenant une source de lumière blanche annulaire (106), des moyens pour disposer ladite source au contact de la peau et des moyens d'analyse de la lumière transmise au travers de la peau (104), lesdits moyens d'analyse de la lumière (104) étant adaptés pour être reliés à un système de traitement informatisé et sont disposés en un endroit se confondant avec l'axe de symétrie de la source de lumière annulaire (106).

2. Diaphanoscope selon la revendication 1 dans lequel lesdits moyens d'analyse de la lumière sont une caméra vidéo.

3. Diaphanoscope selon la revendication 1 ou 2 dans lequel la source de lumière annulaire est disposée à une extrémité d'un élément cylindrique creux, l'autre extrémité du dit élément cylindrique étant fixée aux dits moyens d'analyse de la lumière.

4. Diaphanoscope selon l'une quelconque des revendications précédentes comprenant des moyens pour corriger l'émission de la lumière émise par rapport à l'épaisseur du tissu sous-cutané

5. Diaphanoscope selon l'une quelconque des revendications précédentes comprenant des moyens pour corriger l'émission de la lumière émise par rapport à la pigmentation de la peau.

6. Diaphanoscope selon l'une quelconque des revendications précédentes comprenant des moyens pour exclure des structures correspondant à du sang intravasculaire.

7. Diaphanoscope selon l'une quelconque des revendications précédentes comprenant des moyens pour analyser la densité et la démarcation des ecchymoses.

8. Diaphanoscope selon l'une quelconque des revendications précédentes comprenant des moyens pour faire varier la ou les longueurs d'ondes utilisée(s).

9. Utilisation d'un diaphanoscope tel que défini dans l'une quelconque des revendications précédentes comprenant une étape lors de laquelle on applique une source de lumière blanche de forme annulaire.

10. Utilisation d'un diaphanoscope selon la revendication 9 comprenant une étape où l'on mesure l'épaisseur du tissu sous-cutané préalablement à l'analyse de la lumière transmise à travers la peau et une étape où l'on introduit des données relatives à l'épaisseur de la peau dans le système de traitement informatisé.

11. Utilisation d'un diaphanoscope selon la revendication 9 comprenant une étape où l'on détermine la pigmentation préalablement à l'analyse de la lumière transmise à travers la peau et une étape où l'on introduit des données relatives à la pigmentation de la peau dans le système de traitement informatisé.

12. Utilisation d'un diaphanoscope selon la revendication 9 comprenant une étape où l'on identifie des structures correspondant à du sang intravasculaire.

13. Utilisation d'un diaphanoscope selon la revendication 9 comprenant une étape d'analyse de la densité et de la démarcation des ecchymoses.

14. Méthode d'analyse de la peau par diaphanoscopie pour l'identification d'ecchymoses sous-cutanées invisibles à l'oeil nu, ladite méthode comprenant l'analyse de la lumière blanche qui traverse la peau, **caractérisée par le fait que** l'on applique sur la peau une source de lumière de forme annulaire.
